# FASCICULE DE BREVET EUROPEEN

(11) **EP 1 781 359 B1**
(45) Date de publication et mention de la délivrance du brevet: **07.05.2008**
(21) Numéro de dépôt: 05793388.9
(22) Date de dépôt: 28.07.2005
(51) Int. Cl.: A61M 5/32

(54) **DISPOSITIF DE PROTECTION D'AIGUILLE DESTINE A UNE SERINGUE, ET DISPOSITIF D'INJECTION LE COMPORTANT**
SCHUTZVORRICHTUNG FÜR SPRITZENNADEL UND INJEKTIONSVORRICHTUNG DAMIT
SYRINGE NEEDLE PROTECTIVE DEVICE AND INJECTING DEVICE PROVIDED THEREWITH

(30) Priorité: 27.08.2004 FR 0409163
(43) Date de publication de la demande: 09.05.2007
(73) Titulaire: SEDAT, 69540 Irigny (Rhône) (FR)
(72) Inventeur: PESSIN, Olivier, F-69290 GREZIEU LA VARENNE (FR)
(74) Mandataire: Domenego, Bertrand
(86) Numéro de dépôt international: PCT/FR2005/001983
(87) Numéro de publication internationale: WO 2006/027447

(56) Documents cités:
- WO-A-01/24856
- FR-A- 2 835 753
- FR-A- 2 837 107
- US-A- 5 591 138

## Description

La présente invention concerne un dispositif de protection d'aiguille comportant :
- un support de protecteur délimitant un conduit de réception d'un corps de seringue;
- un protecteur d'aiguille délimitant une ouverture d'extrémité pour le passage de l'aiguille et d'un capuchon de protection de l'aiguille et présentant à son autre extrémité des excroissances radiales formant appui-doigt, lequel protecteur d'aiguille est mobile par rapport au support de protecteur entre une position rétractée et une position déployée ;
- un ressort de compression appliqué entre le support de protecteur et le protecteur d'aiguille ; et
- des moyens de retenue initiale du protecteur d'aiguille à l'encontre de l'action du ressort comprimé, lesquels moyens sont libérables par déplacement du protecteur d'aiguille par rapport au support de protecteur suivant une direction de libération.

L'invention s'applique notamment au domaine des seringues d'injection préremplies à usage unique, destinées en particulier aux injections intramusculaires ou souscutanées.

Ces dispositifs sont destinés à limiter au maximum pour l'utilisateur le risque de piqûre accidentelle post-injection puisqu'une fois l'injection du contenu de la seringue réalisée, l'aiguille de la seringue est retirée du patient et le protecteur est automatiquement amené en position déployée au-delà de l'extrêmité piquante de l'aiguille.

Un tel dispositif de protection est décrit notamment dans le document FR 2 837 107.

Le dispositif décrit dans ce document est tel que le déclenchement de la mise en place du protecteur d'aiguille est assuré par un déplacement axial vers l'arrière du protecteur par rapport au support d'aiguille. Ce déplacement s'effectue à l'encontre du ressort interposé entre le support de protecteur et le protecteur de sorte que la retenue du protecteur d'aiguille pour éviter le déclenchement du dispositif n'est assurée que par la force du ressort.

Les seringues mises en place dans de tels dispositifs de protection sont équipées initialement d'un capuchon amovible de protection de l'aiguille. La seringue ainsi équipée du capuchon est introduite par l'extrémité arrière ou proximale du protecteur. Alors que la seringue est en place dans le dispositif, le capuchon est extrait depuis l'ouverture d'extrémité avant du protecteur.

Les capuchons sont généralement formés en caoutchouc et sont maintenus à l'extrémité de la seringue autour de l'aiguille par l'action élastique radiale du caoutchouc. Ainsi, la force de retenue du capuchon à l'extrémité du corps de seringue peut être relativement importante, nécessitant alors une traction forte sur le protecteur pour permettre son retrait.

Afin de maintenir le dispositif lors du retrait du capuchon, l'utilisateur retient sur le protecteur depuis les excroissances radiales formant appuie-doigt qui sont ménagées sur le protecteur.

Ainsi, lors de la traction sur le capuchon, celui-ci peut entraîner la seringue et le support de protecteur qui est solidaire et déplacer axialement le protecteur par rapport au support de protecteur, ce qui provoque alors le déclenchement du dispositif, alors même que l'injection n'a pas encore été effectuée.

L'invention a pour but de proposer un dispositif de protection d'aiguille dont les risques de déclenchement accidentel lors du retrait du capuchon de protection d'aiguille sont réduits.

A cet effet, l'invention a pour objet un dispositif de protection du type précité, caractérisé en ce qu'il comporte des moyens mécaniques de blocage du protecteur d'aiguille par rapport au support de protecteur suivant la direction de libération, lesquels moyens sont activés sous l'action d'un capuchon de protection de l'aiguille engagé dans l'ouverture d'extrémité de protecteur d'aiguille, sont désactivables par désengagement du capuchon de protection de l'aiguille hors de l'ouverture d'extrémité du protecteur d'aiguille.

Suivant d'autres caractéristiques de ce dispositif, prises isolément ou selon toutes les combinaisons techniquement possibles :
- les moyens mécaniques de blocage comportent au moins une languette déformable élastiquement solidaire d'un premier du protecteur et du support, la ou chaque languette étant déplaçable entre une position de blocage en butée axiale contre le second du protecteur et du support en présence du capuchon et une position de déblocage à l'écart du second du protecteur et du support en l'absence du capuchon, la ou chaque languette s'étendant au repos dans sa position de déblocage et la ou chaque languette présente une plage d'actionnement pour son maintien déformé en position de blocage sous l'action du capuchon engagé dans l'ouverture d'extrémité du protecteur d'aiguille ;
- la ou chaque languette présente une saillie propre à s'engager dans une lumière ménagée dans le second du protecteur et du support ;
- la ou chaque languette est solidaire du protecteur d'aiguille ; et
- la ou chaque languette s'étend dans le prolongement du support de protecteur à son extrémité avant.

L'invention a en outre pour objet un dispositif d'injection comportant, d'une part, une seringue qui comprend un corps tubulaire à l'extrémité distale duquel est fixée une aiguille et un piston d'injection monté à coulissement dans le corps, et d'autre part, un dispositif de protection d'aiguille tel que décrit ci-dessus.

L'invention sera mieux comprise à la lecture de la description qui va suivre, donnée uniquement à titre d'exemple et faite en se référant aux dessins sur lesquels :
- la figure 1 est une vue éclatée en perspective d'un dispositif d'injection selon l'invention avant assemblage ;
- la figure 2 est une vue en élévation du dispositif de protection appartenant au dispositif d'injection de la figure 1, en position rétractée ;
- la figure 3 est une vue en coupe selon le plan III-III indiqué sur la figure 2 ;
- la figure 4 est une vue de dessus selon la flèche IV indiquée sur la figure 2 ;
- la figure 5 est une vue en coupe selon le plan indiqué V-V sur la figure 4;
- la figure 6 est une vue suivant le même plan de coupe que celui de la figure 5, du dispositif d'injection selon l'invention avant utilisation ;
- la figure 7 est une vue de dessus selon la flèche VII indiquée sur la figure 6 ;
- la figure 8 est une vue analogue à celle de la figure 6, le dispositif d'injection étant prêt pour l'utilisation ;
- la figure 9 est une vue identique à celle de la figure 8, le dispositif d'injection ayant son piston tiré en position extrême ;
- la figure 10 est une vue suivant le même plan de coupe que celui de la figure 3, du dispositif d'injection en fin de d'utilisation ;
- la figure 11 est une vue identique à celle de la figure 10, la seringue du dispositif d'injection n'étant pas représentée ;
- la figure 12 est une vue en coupe selon le plan XII-XII indiqué sur la figure 10 ; et
- les figures 13 à 15 illustrent le dispositif d'injection en position déployée, la figure 13 étant une vue analogue à celle de la figure 10, la figure 14 étant une vue en coupe selon le plan XIV-XIV indiqué sur la figure 13 et la figure 15 est une vue identique à celle de la figure 13, la seringue n'étant pas représentée.

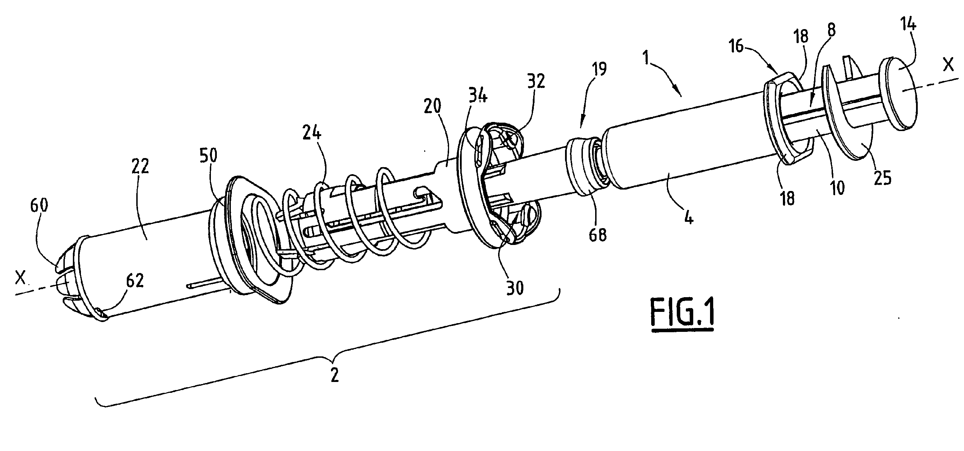

Sur la figure 1 est représenté en perspective un dispositif d'injection formé d'une seringue 1 et d'un ensemble de protection 2. Dans toute la suite, les termes « proximal » et « arrière » sont synonymes, tout comme les termes « distal » et « avant ».

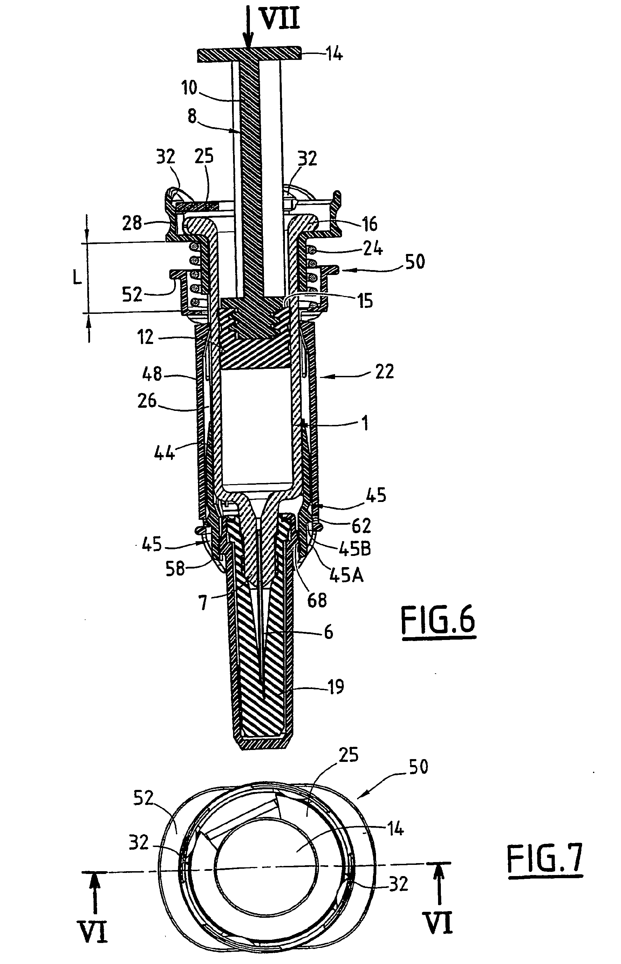

La seringue 1 est une seringue en verre de forme standard, destinée à un usage unique. Elle contient un liquide à injecter, de façon intramusculaire ou sous-cutanée, à un patient. Elle comporte à cet effet un corps 4 et une aiguille 6 visible notamment sur la Figure 6. Cette aiguille est solidaire de l'extrémité distale du corps 4 par un collet 7.

La seringue comporte en outre un piston 8 engagé dans le corps 4. Ce piston comporte classiquement une tige 10 pourvue à son extrémité distale d'un tampon 12 visible sur la figure 6 et d'une tête d'appui 14 sur laquelle est destiné à s'appuyer le pouce de la main du praticien.

La tige 10 a dans sa partie courante une section inférieure à celle du tampon 12 de sorte qu'un épaulement périphérique 15 visible sur la figure 6 est formé sur le tampon 12 autour de la tige 10.

Le corps 4 de la seringue comporte, dans sa partie proximale, un col 16 délimitant circonférentiellement deux oreilles 18 diamétralement opposées, destinées normalement, notamment en l'absence de l'ensemble 2, à former des surfaces d'appui pour l'index et le majeur du praticien lors de la manipulation de la seringue et de l'injection du liquide s'y trouvant.

Initialement, l'aiguille 6 est protégée par un capuchon amovible 19 retenu sur le corps de la seringue en étant engagé sur le collet 7.

L'ensemble de protection 2, d'axe général X-X, comporte essentiellement, comme représenté sur la figure 1 :
- un support 20 de forme générale tubulaire ;
- un manchon protecteur 22 disposé coaxialement au support 20 et de diamètre supérieur à celui du support,
- un ressort 24 ; et
- une épingle 25 de butée du piston et d'accrochage de la seringue dans l'ensemble de protection.

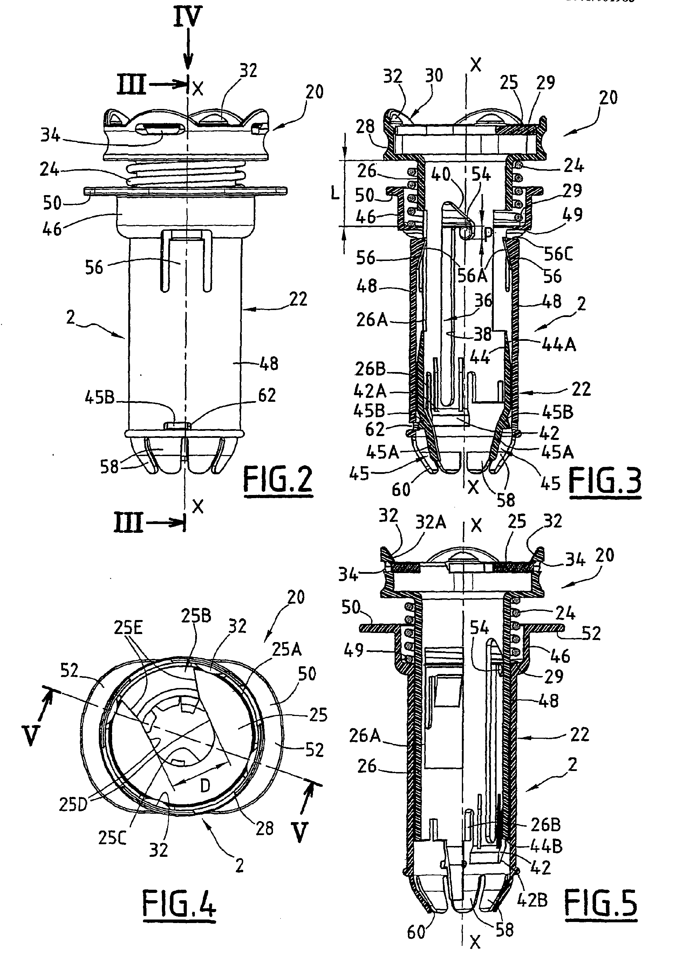

Ces éléments vont successivement être détaillés ci-dessous, en regard des figures 2 à 4.

Le support 20 comporte un tronçon principal 26, sensiblement cylindrique et de diamètre interne sensiblement égal au diamètre externe du corps 4 de la seringue. Ce tronçon 26 présente deux évidements longitudinaux 26A qui sont diamétralement opposés. Le tronçon 26 se prolonge à son extrémité proximale par un tronçon secondaire 28 de plus grands diamètres intérieur et extérieur que ceux du tronçon principal 26, en formant un épaulement radial 29. Le tronçon 28 présente un diamètre intérieur supérieur au diamètre maximal du col 16 de la seringue.

Le tronçon proximal 28 est pourvu de moyens 30 de retenue de l'épingle 25 pour que celle-ci enserre le col de seringue 16 entre l'épaulement 29 et l'épingle 25 comme illustré sur la figure 6.

L'épingle 25 visible sur la Figure 4 est plane et présente une forme de disque évidé pour créer pour former un U. Elle est délimitée à sa périphérie par un contour circulaire 25A de diamètre très légèrement inférieur au diamètre intérieur du tronçon secondaire 28. Elle présente une encoche 25B s'engageant dans le disque formant l'épingle au-delà du centre de celui-ci. Cette encoche 25B débouche radialement à la périphérie du disque formant l'épingle. Elle présente un fond semi-circulaire 25C et deux rives parallèles 25D prolongeant le fond 25C. Ces rives 25D sont prolongées jusqu'à la périphérie 25A du disque par des pans divergeant 25E de sorte que l'encoche 25B s'évase au voisinage de son extrémité débouchante.

Le diamètre du fond 25C et la distance séparant les rives 25D, tous deux désignés par D, sont choisis supérieurs au diamètre de la partie courante de la tige de piston 10, et inférieurs au diamètre du tampon 12 prolongeant la tige 10. Ainsi, ce diamètre D est inférieur à la section intérieure du corps de seringue 4.

Les moyens de retenue 30 comportent des crochets déformables 32 diamétralement opposés qui sont portés par le tronçon 28 du support de protecteur. Ces crochets sont visibles notamment sur les figures 1 et 5. La distance séparant les crochets diamétralement opposés est inférieure au diamètre extérieur de l'épingle 25. Des lumières 34 sont ménagées dans le tronçon 28 le long des crochets 32 pour conférer à ceux-ci une élasticité.

Chacun de ces crochets ménage une rampe 32A sensiblement tronconique s'évasant vers l'extrémité libre du tronçon 28. Ces rampes 32A sont destinées à permettre de repousser élastiquement les crochets vers l'extérieur sous l'action de l'épingle 25 lors de la fixation du support 20 sur la seringue 1. Les crochets 32 sont distants de l'épaulement 29 d'une distance sensiblement égale aux épaisseurs cumulées des oreilles 18 et de l'épingle 25. Les crochets 32 forment ainsi un moyen de clipsage de l'épingle 25 pour la retenue du col de seringue 16.

Deux rainures traversantes 36 en forme de crosse sont ménagées l'une en face de l'autre dans le tronçon principal 26. Chaque rainure est constituée d'une première partie rectiligne 38 qui s'étend sensiblement suivant l'axe X-X du support 20 sur une longueur supérieure à celle de l'aiguille 6, et d'une seconde partie rectiligne 40 qui s'étend de manière inclinée par rapport au même axe X-X. La partie inclinée 40 débouche à l'extrémité proximale de la première partie rectiligne 38, en formant un V dont la pointe est dirigée du côté proximal de l'ensemble 2.

Le tronçon principal 26 comporte à son extrémité distale une première paire de languettes élastiques 42 diamétralement opposées, situées chacune dans le prolongement des rainures 36 (figure 5). Ces languettes 42 présentent une face interne 42A de forme sensiblement cylindrique et une face externe 42B sensiblement tronconique, divergente vers l'arrière.

Le tronçon principal 26 comporte une paire de rampes externes 44 diamétralement opposées, situées entre les languettes élastiques 42 en suivant la circonférence de l'extrémité distale de ce tronçon. Elles présentent une surface externe inclinée 44A sensiblement tronconique, divergente vers l'avant, et une surface distale 44B sensiblement plane. Les surfaces externes 44A sont ainsi tournées vers les évidements 26A.

Les évidements longitudinaux 26A sont ménagés dans le tronçon principal 26 (figure 3) à l'extrémité proximale de ces rampes 44.

Par ailleurs, de part et d'autre des languettes 42, des fentes axiales 26B sont prévues à partir de l'extrémité distale du support, de sorte que, avant insertion du corps 4 de la seringue 1 à l'intérieur du support 20, ces languettes 42 sont radialement déformables, notamment vers l'intérieur.

A son extrémité distale ou avant, le tronçon principal 26 présente, entre les languettes élastiques 42, deux autres languettes 45 de solidarisation axiale du protecteur 22 et du support de protecteur 20 en présence du capuchon de protection 19 placé sur le corps de seringue.

Plus précisément et comme illustré sur la Figure 3, ces languettes comportent chacune une jambe 45A prolongeant le tronçon principal 26 et venue de matière avec celui-ci. Chaque jambe 45A porte extérieurement une saillie 45B propre à s'engager dans une lumière complémentaire ménagée dans le protecteur.

Les saillies 45B sont prévues dans la moitié de la longueur des jambes 45A s'étendant depuis la région de liaison des jambes au tronçon principal 26. Ainsi, les jambes 45A se prolongent au-delà des saillies 45B.

Comme illustré sur la Figure 3, les languettes sont, au repos, inclinées vers l'axe X-X du support, c'est-à-dire qu'elles convergent l'une vers l'autre en direction de leur extrémité libre et qu'elles s'étendent dans le prolongement du passage intérieur délimité par le support de protecteur.

Ces languettes 45 sont déformables élastiquement vers l'extérieur de sorte que, en position de repos, les saillies sont complétement contenues dans le prolongement de l'encombrement général du tronçon principal 26 alors que, en position déformée, les saillies 45B s'étendent radialement vers l'extérieur au-delà du prolongement de l'encombrement général du tronçon principal 26.

La distance séparant les extrémités libres des deux languettes 45, lorsqu'elles sont dans leur position de repos est inférieure au diamètre extérieure minimal de la bague de retenue du capuchon protecteur 19 de seringue.

Le manchon protecteur 22 est de longueur sensiblement égale à celle du corps 4 de la seringue. II est constitué de deux tronçons cylindriques 46 et 48, le tronçon proximal 46 étant de diamètre légèrement supérieur à celui du tronçon principal 48. Ces deux tronçons se raccordent en formant un épaulement radial 49.

Le manchon 22 comporte solidairement, dans sa partie proximale, une collerette extérieure 50 sous forme de deux oreilles 52 diamétralement opposées (figures 4 et 5).

Egalement dans sa partie proximale, mais à l'intérieur du manchon protecteur 22, deux tétons 54 diamétralement opposés sont solidaires du manchon (figure 5). Ces deux tétons sont reçus et guidés dans respectivement les deux rainures 36 du support. Le support et le manchon sont ainsi mobiles l'un par rapport à l'autre en translation suivant leur axe commun et en rotation limitée autour du même axe lorsque les tétons se trouvent dans les parties inclinées 40. Les parties inclinées 40 forment alors des poches de rétention des tétons 54, ces poches ayant une profondeur de rétention notée p sur la Figure 3. Cette profondeur est mesurée suivant l'axe du protecteur.

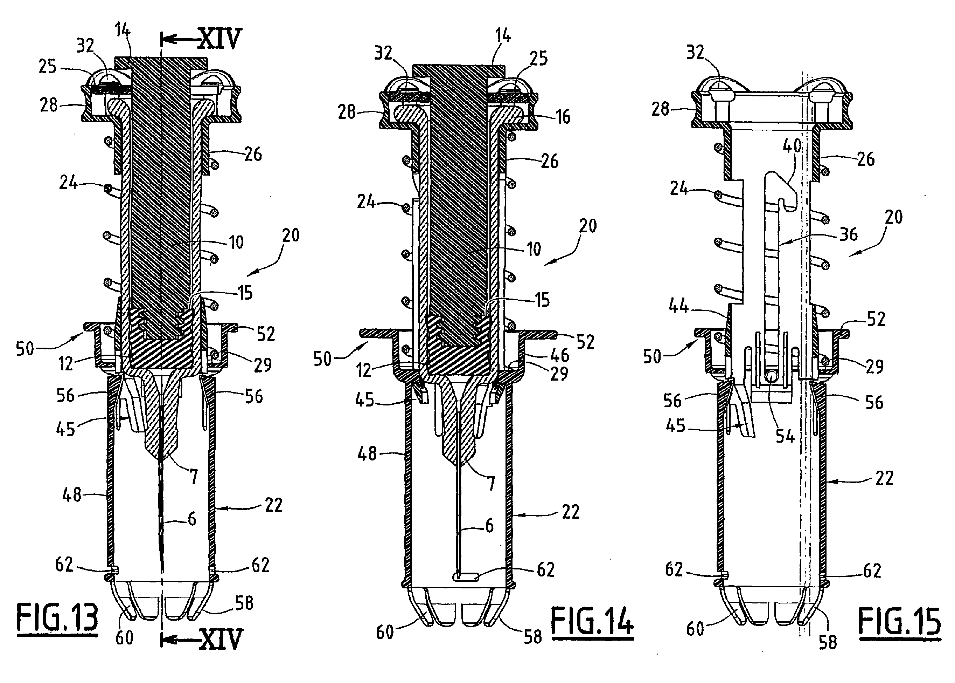

Le support 20 et le manchon 22 sont mobiles entre une position rétractée du manchon, dans laquelle l'essentiel du manchon recouvre l'essentiel du support et les tétons 54 sont situés à l'extrémité distale de chacune des parties de rainures inclinées 40, comme représenté sur les figures 2 à 8, et une position déployée du manchon dans laquelle celui-ci s'étend axialement en saillie du support et les tétons sont situés à l'extrémité distale de la partie de rainure rectiligne 38, comme représenté sur les figures 13 à 15.

Lorsque la seringue 1 est fixée sur l'ensemble 2, ces positions extrêmes correspondent respectivement à une configuration d'injection dans laquelle l'aiguille 6 de la seringue 1 est dégagée et destinée à être insérée dans un patient, et à une configuration de protection dans laquelle cette aiguille est entourée du manchon protecteur 22.

La partie proximale du manchon 22 comporte en outre intérieurement une paire de crochets longitudinaux déformables 56 diamétralement opposés. Ces crochets sont délimités dans le manchon 22 par des fentes latérales. Ils sont liés au manchon à leur extrémité distale. Chaque crochet présente à son extrémité libre proximale une protubérance intérieure.

En l'absence de seringue, et comme illustré sur la Figure 3, les surfaces externes des crochets 56 s'étendent dans le prolongement du manchon. Au contraire, les protubérances internes de ces crochets font saillie à l'intérieur du passage cylindrique délimité par le manchon 22. Chaque protubérance présente une face avant 56A de forme sensiblement tronconique s'écartant vers l'avant de l'axe du manchon 22. Ces faces avant 56A sont ainsi tournées vers l'extrémité avant du protecteur.

Chaque face avant 56A est adaptée pour coopérer avec une surface inclinée 44A formée par les rampes 44 du support.

A son extrémité libre, chaque crochet 56 présente un front transversal incliné 56C formant butée.

Dans la position rétractée du manchon, les crochets 56 s'étendent à l'intérieur des évidements 26A ménagés dans le support 20. Dans la position déployée du manchon, comme représenté sur la figure 13, les faces d'extrémité 56C des crochets 56 sont en butée axiale contre les languettes 42, les crochets et languettes formant de la sorte un ensemble de blocage rigide en position déployée.

Le manchon 22 est par ailleurs pourvu à son extrémité distale d'une couronne de languettes déformables 58, dont les bords distaux forment une ouverture 60 sensiblement circulaire, de diamètre inférieur au diamètre intérieur du tronçon principal 26 du support 20.

Enfin, à son extrémité distale, le tronçon principal 48 du manchon protecteur comporte deux lumières oblongues traversantes 62 propres à recevoir les saillies 45B portées par la seconde paire de languettes 45 lorsque celles-ci sont déformées sous l'action de la bague du capuchon 19 de protection d'aiguille engagé sur une seringue contenue dans le dispositif.

En l'absence de capuchon, alors que les languettes 45 sont dans leur position de repos, les saillies 45B sont totalement hors des lumières 62 permettant un déplacement libre du protecteur par rapport au support.

Le ressort 24 est un ressort spiralé, disposé entre le manchon protecteur 22 et le support de protecteur 20. Plus précisément, le ressort est logé entre l'épaulement 29 du support 20 et l'épaulement 49 du manchon 22.

Dans la position rétractée du manchon, le ressort 24 est dans un état comprimé, possédant ainsi une énergie de décompression liée à la raideur du ressort et à la différence entre la longueur du ressort à l'état au repos et sa longueur, notée L sur les figures 2 à 8, à l'état comprimé. Cela revient à dire que le ressort 24 présente un seuil de force de compression supplémentaire qui correspond à la force minimale nécessaire pour comprimer davantage le ressort à partir de son état comprimé initial des figures 2 à 8. La raideur du ressort et/ou la longueur de compression initiale L sont choisies de telle sorte que ce seuil de force soit supérieur à l'effort de poussée nécessaire pour déplacer le piston 8 de la seringue 1 sur toute sa course d'injection. Plus précisément, la force du ressort à l'état verrouillé est supérieure à la somme de l'effort d'injection, c'est-à-dire d'évacuation du liquide à l'extérieur de l'aiguille 6 de la seringue 1, et des efforts de décollement et de glissement du poussoir 12 à l'intérieur du corps 4 de la seringue.

Le capuchon 19 est de forme générale tubulaire, et est représenté sur les figures 1 et 6.

II est adapté pour entourer l'aiguille 6 avant utilisation de la seringue 1. Ce capuchon est fermé à une de ses extrémités et son extrémité opposée est formée par une bague annulaire 68 de diamètre extérieur adapté pour à la fois être conjugué de la surface 42A des languettes 42 et être supérieur au diamètre de l'ouverture 60 formée par les languettes 58 du manchon protecteur 22. La face intérieure de cette bague 68 est destinée à adhérer sur le collet en verre 7 du corps de seringue où est fixée l'aiguille 6, notamment pour garantir une certaine étanchéité aux bactéries.

En outre, le diamètre extérieur de la bague 68 du capuchon est supérieur à la distance séparant au repos les extrémités libres des languettes 45.

Le fonctionnement du dispositif d'injection selon l'invention est le suivant.

Le dispositif de protection 2 est assemblé dans sa configuration rétractée, c'est-à-dire celle des figures 2 à 8. Le manchon protecteur 22 est pour cela enfilé autour du support 20 à partir de l'extrémité distale du support, en disposant entre eux le ressort 24. Plus précisément, on déplace axialement vers l'arrière le manchon 22 par rapport au support, tout en déformant radialement vers l'extérieur les crochets 56 au moyen d'un outil adapté, et ce au moins jusqu'à ce qu'ils atteignent axialement la partie avant des évidements longitudinaux 26A.

Puis toujours en déplaçant le manchon vers l'arrière, les tétons 54 sont appliqués contre les surfaces externes 42B des languettes 42, en déformant ces dernières vers l'intérieur, jusqu'à ce que les pions soient reçus dans les parties rectilignes 38. Le déplacement du protecteur 22 vers l'arrière se poursuit ensuite jusqu'à ce que les tétons 54 soient reçus dans les parties de rainures inclinées 40, en faisant pivoter l'un par rapport à l'autre le support et le protecteur. Le protecteur se retrouve ainsi en position rétractée.

La seringue en verre 1 est préremplie d'un liquide à injecter dans un patient. Cette seringue est équipée du capuchon 19 qui enserre le collet 9 du corps de seringue 4.

La seringue équipée du capuchon 19 est insérée à l'intérieur de l'ensemble 2 pour former le dispositif d'injection, tel que représenté sur les figures 6 et 7. Plus précisément, le corps 4 de la seringue est déplacé sensiblement axialement à l'intérieur du support 20.

Le déplacement du corps de seringue dans le protecteur est effectué jusqu'à ce que le col de seringue 16 s'appuie sur l'épaulement 29.

Dans cette position, l'épingle 25 est alors rapportée sur le col 16. A cet effet, si la tige de piston 10 est déjà montée sur le tampon 12, l'épingle 25 est engagée autour de la tige de piston, celle-ci se trouvant dans l'encoche. L'épingle 25 est déplacée suivant la longueur de la tige 10 puis est engagée dans le tronçon principal 28 en déformant radialement vers l'extérieur les crochets 32 de manière à assurer un enclenchement élastique de l'épingle.

Les oreilles 18 du col de seringue 16 sont alors retenues axialement par l'épingle 25 elle-même retenue par les crochets 32.

Dans la mesure où le col de seringue 16 est totalement enclipsé à l'intérieur du tronçon 28, il ne peut plus remplir son rôle habituel de formation de surface d'appui pour l'index et le majeur du praticien. Cette fonction d'appui est réalisée par la collerette 50 solidaire du manchon 22. En effet, dans la mesure où la longueur du manchon protecteur 22 est sensiblement égale à celle du corps de seringue 4 et/ou où la collerette 50 est ménagée au niveau de l'extrémité proximale de ce manchon, le praticien peut alors manipuler la seringue en faisant reposer son pouce sur la tête d'appui 14 du piston 8 comme à l'accoutumée, et en faisant reposer son index et son majeur sur les faces des oreilles 52 dirigées vers l'aiguille 6.

Par ailleurs, lorsque la seringue 1 est fixée sur le support de protecteur 20 comme représenté sur les figures 6 et 7, le capuchon 19 s'étend en saillie à l'extérieur du manchon protecteur 22.

Qui plus est, le diamètre extérieur élargi de la bague 68 du capuchon se trouve engagé entre les extrémités libres des languettes 45, ce qui assure ainsi leur déformation radiale vers l'extérieur. Sous l'action de cette déformation, les saillies 45B sont reçues dans les lumières 62. Dans cette position, la coopération des saillies 45B portées par le support de protecteur et des lumières 62 ménagées dans le protecteur assure une solidarisation axiale du protecteur et du support, interdisant ainsi une libération du ressort, le téton 54 ne pouvant quitter l'extrémité de la crosse dans laquelle il est engagé.

Lorsque le praticien est prêt à réaliser l'injection du liquide contenu dans la seringue, il retire le capuchon 19 en le tirant axialement vers l'avant.

Pour retirer le capuchon, le praticien peut maintenir la seringue en retenant le protecteur d'une main depuis les surfaces d'appui 50 et tirer sur le capuchon 19 avec l'autre main. Même si la résistance au retrait du capuchon est très importante, la liaison axiale positive assurée par les saillies 45B engagées dans les lumières 62 évite que le ressort 24 ne puisse être comprimé et que les tétons 54 puissent être dégagés hors de la crosse de retenue.

Ainsi, aucun déclenchement accidentel du protecteur d'aiguille ne peut avoir lieu lors du retrait du capuchon.

En revanche, après retrait du capuchon comme illustré sur la figure 7, le protecteur 22 peut être déplacé par rapport au support 20 puisque les languettes 45 reprennent leur position de repos sous l'action de leur élasticité et les saillies 45A sont alors dégagées des lumières 62.

Après le décollement de la bague 68 du collet 9, la bague 68 franchit l'ouverture 60 en déformant les languettes 58. Une fois le capuchon 19 retiré, les languettes 58 reprennent leur position initiale.

Le praticien peut alors être amené à pratiquer une opération connue sous l'expression « test veine ». Celle-ci consiste à vérifier que la seringue a été correctement piquée dans un muscle du patient et non dans une artère ou une veine. A cet effet, le piston 8 est tiré vers l'extrémité proximale. Si du sang est aspiré dans le corps de seringue, le praticien déduit que l'aiguille est piquée dans une veine ou une artère et il recommence alors la mise en place de l'aiguille.

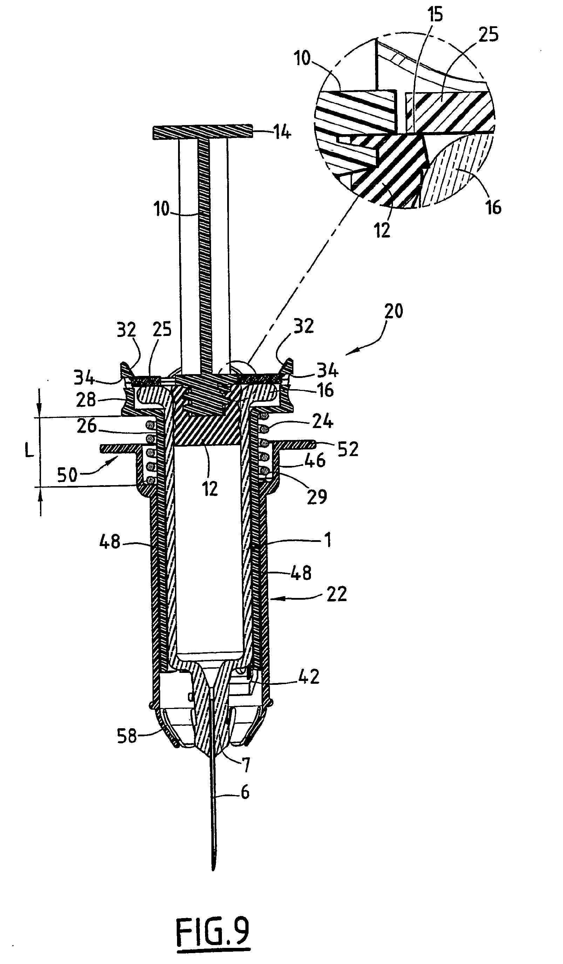

En cas de traction excessive exercée par le praticien sur la tige de piston, le tampon 12 est arrêté dans son déplacement en butant par l'intermédiaire de l'épaulement 15 contre l'épingle 25 comme illustré sur la figure 9. En effet, l'encoche 25B définit un passage de section inférieure à la section du tampon 12 de sorte que celui-ci ne peut passer au-delà de l'épingle. En revanche, la tige de piston 10 ayant un diamètre inférieur, celui-ci peut coulisser librement au travers de l'encoche 25B.

Ainsi, on conçoit que l'épingle 25 assure à la fois la retenue du corps de seringue 4 dans le protecteur et d'autre part constitue une butée d'arrêt du tampon 12 en cas de traction excessive sur celui-ci. En l'absence de l'épingle, le tampon pourrait être extrait du corps de seringue, rendant ainsi le dispositif d'injection inutilisable.

L'arrêt du piston en cas de traction excessive sur celui-ci est également utile lors du remplissage de la seringue au travers de l'aiguille par exemple lors d'une reprise de lyophilisat.

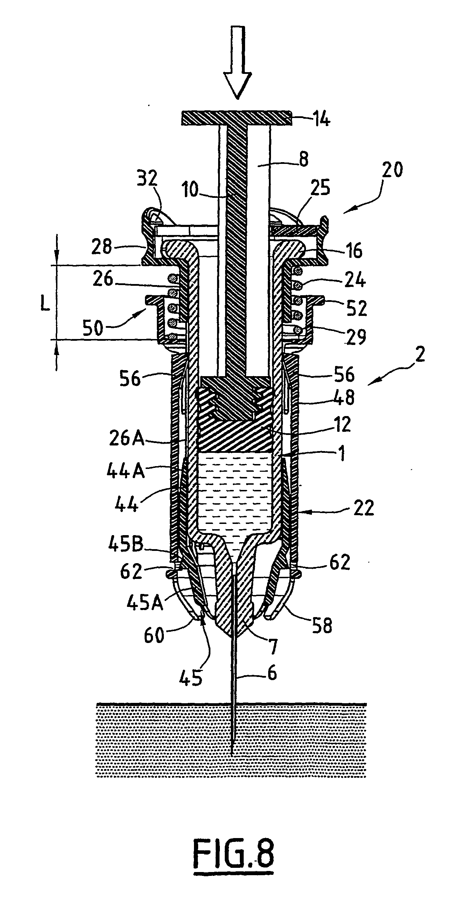

Pour procéder à l'injection proprement dite, le praticien expulse le liquide contenu dans la seringue en exerçant une poussée sur la tête d'appui 14 du piston, son index et son majeur demeurant en contact avec les faces dirigées vers l'aiguille des oreilles 52. Durant l'injection, aucun mouvement ne se produit entre le support de protecteur 20 et le manchon protecteur 22, le ressort 24 demeurant comprimé avec une longueur L, comme représenté sur la figure 8.

L'injection se poursuit jusqu'à ce que le poussoir 12 du piston 8 parvienne en fin de course d'injection.

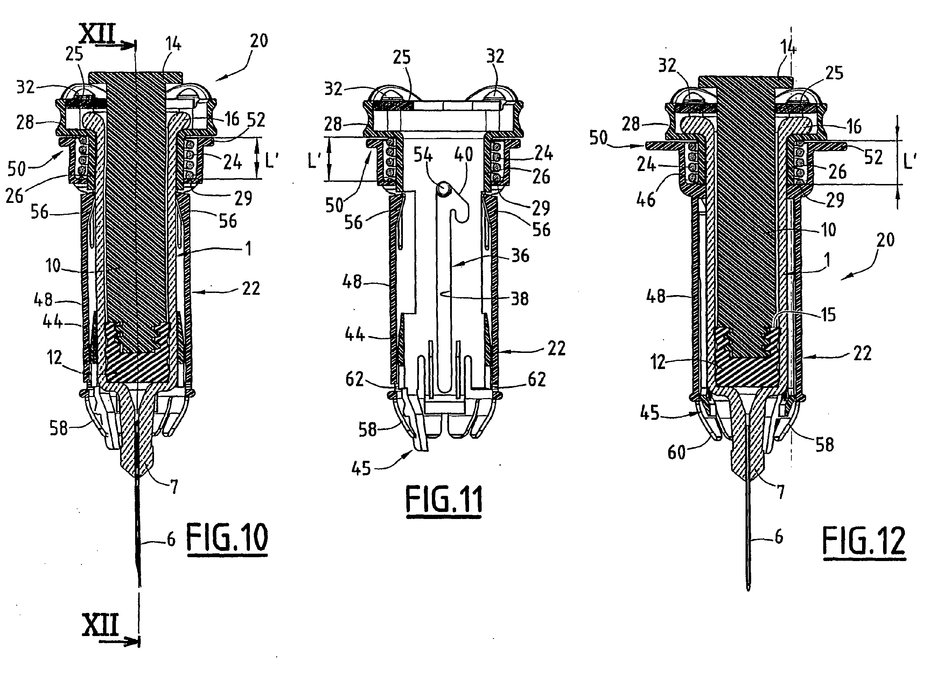

Le praticien retire alors l'aiguille du patient. Pour déclencher l'ensemble de protection 2, le praticien exerce une pression supplémentaire sur la tige de piston 8. Cette pression doit être supérieure à la force prédéterminée produite par le ressort 24 à l'état verrouillé, de sorte que ce ressort est davantage comprimé et passe de sa longueur L à une longueur L' plus petite, comme représenté sur les figures 10 à 12. Pour ce faire, en raisonnant à support de seringue immobile, le manchon protecteur 22 se déplace axialement vers l'extrémité proximale du support 20. Le praticien réalise ce mouvement en exerçant une pression correspondante au moyen de son index et de son majeur sur les oreilles 52 de la collerette 50 du manchon 22. Cette pression entraîne, de façon combinée au mouvement de translation, la rotation du manchon protecteur 22 autour du support de seringue 20, les tétons 54 étant guidés par les parties de rainure inclinées 40. Ce mouvement de rotation se poursuit jusqu'à ce que les tétons atteignent l'extrémité proximale de cette partie de rainure 40, c'est-à-dire l'extrémité proximale de la partie de rainure longitudinale 38, comme visible sur la figure 11. Le dispositif 2 est alors en position de déverrouillage du ressort 24.

Le praticien relâche ensuite la pression qu'il exerçait jusqu'alors sur la collerette 50, permettant au ressort 24 de se détendre jusqu'à un état de repos. Les tétons 54 se déplacent en translation à l'intérieur de la partie de rainure longitudinale 38, jusqu'à l'extrémité distale de celle-ci comme représenté sur les figures 13 à 15. Le mouvement de translation du manchon protecteur 22 par rapport au support 20 est contrôlable par le praticien, si ce dernier relâche progressivement la retenue digitale qu'il exerce la collerette 50. Lorsque les tétons 54 sont arrivés à l'extrémité distale de la rainure 36 (figure 15), le protecteur est dans sa position déployée.

Par ailleurs, lorsque le manchon protecteur 22 est en mouvement de translation par rapport au support 20, les crochets 56 empruntent les évidements longitudinaux 26A du support, jusqu'à ce qu'ils glissent le long des rampes distales 44 du support, par coopération de leurs surfaces complémentaires 56A et 44A.

En position déployée du protecteur, les crochets 56 sont maintenus par coopération des surfaces 56C et 44B, de sorte que le manchon protecteur 22 ne peut pas être ramené dans sa position initiale. De même, le manchon 22 ne peut pas être facilement arraché du support 20 puisque les tétons 54 sont en butée contre le fond distal de la partie de rainure longitudinale 38 (figure 15), les languettes 42 formant ce fond étant radialement maintenues entre le corps de la seringue 1 et le manchon protecteur 22.

Le dispositif d'injection selon l'invention est ainsi simple d'utilisation, tout en permettant au praticien de contrôler le mouvement de recouvrement de l'aiguille par le manchon protecteur. Le nombre de pièces dont est constitué l'ensemble de protection 2 représenté est réduit à trois.

Le dispositif selon l'invention est adaptable à différents types de seringues, tant au niveau des formes qu'à celui des volumes. Ce dispositif présente donc l'avantage de ne pas remettre en cause la forme générale des seringues utilisées et par conséquent n'entraîne aucune modification des procédés industriels de remplissage de ces seringues.

Diverses variantes du dispositif selon l'invention sont envisageables :
- à la différence de l'exemple de réalisation décrit ci-dessus, les tétons 54 et/ou la collerette 50 du manchon protecteur 22 peuvent être rapportés sur le manchon 22 et non réalisés d'un seul tenant avec celui-ci ;
- à l'inverse du dispositif décrit, les tétons 54 peuvent être réalisés sur la surface externe du support de protecteur 20 et la rainure de guidage 36 ménagée dans le manchon protecteur 22 ; et/ou
- le support 20 peut être réalisé d'une seule pièce avec le corps de seringue 4.

## Revendications

1. Dispositif (2) de protection d'aiguille comportant :
- un support de protecteur (20) délimitant un conduit de réception d'un corps de seringue (4) ;
- un protecteur d'aiguille (22) délimitant une ouverture d'extrémité (60) pour le passage de l'aiguille (6) et d'un capuchon (19) de protection de l'aiguille et présentant à son autre extrémité des excroissances radiales (50) formant appui-doigt, lequel protecteur d'aiguille (22) est mobile par rapport au support de protecteur (20) entre une position rétractée et une position déployée ;
- un ressort de compression (24) appliqué entre le support de protecteur (20) et le protecteur d'aiguille (22) ; et
- des moyens (38, 40, 54) de retenue initiale du protecteur d'aiguille (22) à l'encontre de l'action du ressort comprimé (24), lesquels moyens (38, 40, 54) sont libérables par déplacement du protecteur d'aiguille (22) par rapport au support de protecteur (20) suivant une direction de libération,
**caractérisé en ce qu'**il comporte des moyens mécaniques (45, 62) de blocage du protecteur d'aiguille (22) par rapport au support de protecteur (20) suivant la direction de libération, lesquels moyens sont activés sous l'action d'un capuchon (19) de protection de l'aiguille engagé dans l'ouverture d'extrémité (60) de protecteur d'aiguille (22), et sont désactivables par désengagement du capuchon (19) de protection de l'aiguille hors de l'ouverture d'extrémité (60) du protecteur d'aiguille (22).

2. Dispositif (2) de protection d'aiguille suivant la revendication 1, **caractérisé en ce que** les moyens mécaniques de blocage comportent au moins une languette déformable élastiquement (45) solidaire d'un premier du protecteur (22) et du support (20), la ou chaque languette (45) étant déplaçable entre une position de blocage en butée axiale contre le second du protecteur (22) et du support (20) en présence du capuchon (19) et une position de déblocage à l'écart du second du protecteur (22) et du support (20) en l'absence du capuchon (19), la ou chaque languette (45) s'étendant au repos dans sa position de déblocage et **en ce que** la ou chaque languette (45) présente une plage d'actionnement (45A) pour son maintien déformé en position de blocage sous l'action du capuchon (19) engagé dans l'ouverture d'extrémité (60) du protecteur d'aiguille (22).

3. Dispositif (2) de protection d'aiguille suivant la revendication 2, **caractérisé en ce que** la ou chaque languette (45) présente une saillie (45B) propre à s'engager dans une lumière (62) ménagée dans le second du protecteur (22) et du support (20).

4. Dispositif (2) de protection d'aiguille suivant l'une quelconque des revendications 2 et 3, **caractérisé en ce que** la ou chaque languette (45) est solidaire du protecteur d'aiguille (22).

5. Dispositif (2) de protection d'aiguille suivant la revendication 4, **caractérisé en ce que** la ou chaque languette (45) s'étend dans le prolongement du support de protecteur (20) à son extrémité avant.

6. Dispositif d'injection comprenant, d'une part, une seringue (1) qui comprend un corps tubulaire (4) à l'extrémité distale duquel est fixée une aiguille (6), et un piston d'injection (8) monté à coulissement dans le corps (4) et, d'autre part, un dispositif (2) de protection d'aiguille selon l'une quelconque des revendications précédentes.

## Claims

1. Needle protecting device (2) comprising:
- a protector support (20) defining a receiving channel for a syringe body (4);
- a needle support (22) defining an end opening (60) through which the needle (6) and a protective cap (19) for the needle can pass and having at its other end radial extensions (50) forming a finger rest, said needle protector (22) being movable relative to the protector support (20) between a retracted position and a deployed position;
- a compression spring (24) applied between the protector support (20) and the needle protector (22); and
- means (38, 40, 54) for initially holding the needle protector (22) counter to the action of the compressed spring (24), said means (38, 40, 54) being releasable by moving the needle protector (22) relative to the protector support (20) in a direction of release,
**characterised in that** it comprises mechanical means (45, 62) for blocking the needle protector (22) relative to the protector support (20) in the direction of release, said means being activated under the effect of a protective cap (19) for the needle which engages in the end opening (60) of the needle protector (22), and being capable of being deactivated by disengaging the protective cap (19) for the needle from the end opening (60) of the needle protector (22).

2. Needle protecting device (2) according to claim 1, **characterised in that** the mechanical blocking means comprise at least one elastically deformable tab (45) firmly attached to a first one of the protector (22) and support (20), the or each tab (45) being displaceable between a blocking position axially abutting on the second one of the protector (22) and support (20), in the presence of the cap (19), and an unblocking position at a spacing from the second one of the protector (22) and the support (20), in the absence of the cap (19), the or each tab (45) extending in the unblocking position when at rest, and **in that** the or each tab (45) has an actuating range (45A) for holding it deformed in the blocking position under the effect of the cap (19) engaging in the end opening (60) of the needle protector (22).

3. Needle protecting device (2) according to claim 2, **characterised in that** the or each tab (45) has a projection (45B) capable of engaging in an opening (62) formed in the second one of the protector (22) and support (20).

4. Needle protecting device (2) according to any one of claims 2 and 3, **characterised in that** the or each tab (45) is firmly attached to the needle protector (22).

5. Needle protecting device (2) according to claim 4, **characterised in that** the or each tab (45) extends as a continuation of the protector support (20) at its front end.

6. Injection device comprising, on the one hand, a syringe (1) that comprises a tubular body (4) at the distal end of which is fixed a needle (6), and an injection plunger (8) mounted to slide within the body (4) and, on the other hand, a needle protecting device (2) according to any one of the preceding claims.

## Patentansprüche

1. Vorrichtung (2) zum Schutz einer Nadel, mit:
- einem Nadelschutzträger (20), der einen Kanal zur Aufnahme eines Spritzenkörpers (4) bildet,
- einem Nadelschutz (22), der an einem Ende eine Öffnung (60) für den Durchtritt der Nadel (6) und einer Schutzkappe (19) für die Nadel bildet und an seinem anderen Ende radiale Ausstülpungen (50) bildet, die eine Fingerstütze bilden, welcher Nadelschutz (22) relativ zu dem Nadelschutzträger (20) zwischen einer zurückgzogenen Position und einer ausgefahrenen Position beweglich ist,
- einer zwischen dem Nadelschutzträger (20) und dem Nadelschutz (22) eingefügten Druckfeder (24) und
- Mitteln (38, 40, 54) zum anfänglichen Halten des Nadelschutzes (22) entgegen der Wirkung der komprimierten Feder (24), welche Mittel (38, 40, 54) durch Verschiebung des Nadelschutzes (22) relativ zu dem Nadelschutzträger (20) in einer Freigaberichtung in ihrer Wirkung aufhebbar sind,
**dadurch gekennzeichnet, daß** sie mechanische Mittel (45, 62) zum Blockieren des Nadelschutzes (22) relativ zu dem Nadelschutzträger (20) in der Freigaberichtung aufweist, welche Mittel durch die Wirkung einer Schutzkappe (19) für die Nadel aktivierbar sind, die in die Öffnung (60) am Ende des Nadelschutzes (22) eingreift, und durch Herauslösen der Schutzkappe (19) aus der Öffnung (60) am Ende des Nadelschutzes (22) deaktivierbar sind.

2. Vorrichtung (2) zum Schutz einer Nadel gemäß Anspruch 1, **dadurch gekennzeichnet, daß** die mechanischen Blockagemittel wenigstens eine elastisch verformbare Zunge (45) aufweisen, die mit einem ersten der Bauelemente Nadelschutz (22) und Nadelschutzträger (20) verbunden ist, wobei die oder jede Zunge (45) zwischen einer Blockierstellung in axialer Anlage an dem zweiten der Bauteile Nadelschutz (22) und Nadelschutzträger (20) bei Anwesenheit der Schutzkappe (19) und einer von dem zweiten der Bauteile Nadelschutz (22) und Nadelschutzträger (20) abgerückten Deblockierstellung bei Abwesenheit der Schutzkappe (19) verstellbar sind, wobei die oder jede Zunge (45) sich in Ruhestellung in ihrer Deblockierstellung befindet, und daß die oder jede Zunge (45) einen Betätigungsbereich (45A) aufweist, der dazu dient, sie unter der Wirkung der in die Öffnung (60) am Ende des Nadelschutzes (22) eingreifenden Schutzkappe (19) deformiert in der Blockierstellung zu halten.

3. Vorrichtung (2) zum Schutz einer Nadel nach Anspruch 2, **dadurch gekennzeichnet, daß** die oder jede Zunge (45) einen Vorsprung (45B) aufweist, der dazu geeignet ist, in eine in dem zweiten der Bauteile Nadelschutz (22) und Nadelschutzträger (20) gebildete Öffnung (62) einzugreifen.

4. Vorrichtung (2) zum Schutz einer Nadel nach einem der Ansprüche 2 und 3, **dadurch gekennzeichnet, daß** die oder jede Zunge (45) mit dem Nadelschutz (22) verbunden ist.

5. Vorrichtung (2) zum Schutz einer Nadel nach Anspruch 4, **dadurch gekennzeichnet, daß** die oder jede Zunge (45) sich in Verlängerung des Nadelschutzträgers (20) an seinem vorderen Ende erstreckt.

6. Injektionsvorrichtung mit, einerseits, einer Spritze (1), die einen rohrförmigen Körper (4), an dessen distalem Ende eine Nadel (6) befestigt ist, und einen gleitend in dem Körper (4) montierten Injektionskolben (8) aufweist, und, andererseits einer Vorrichtung (2) zum Schutz der Nadel nach einem der vorstehenden Ansprüche.
